(19) Europäisches Patentamt
European Patent Office
Office européen des brevets

(11) EP 2 493 382 B1

(12) EUROPEAN PATENT SPECIFICATION

(45) Date of publication and mention
of the grant of the patent:
**18.07.2018 Bulletin 2018/29**

(51) Int Cl.:
*A61B 6/03* (2006.01)     *G01T 1/00* (2006.01)
*G06T 11/00* (2006.01)     *G06F 19/00* (2018.01)
*H04N 19/91* (2014.01)     *H04N 19/85* (2014.01)
*A61B 6/00* (2006.01)

(21) Application number: **09749270.6**

(22) Date of filing: **29.10.2009**

(86) International application number:
**PCT/US2009/062567**

(87) International publication number:
**WO 2011/053296 (05.05.2011 Gazette 2011/18)**

(54) **THREE-DIMENSIONAL SCANNER DATA COMPRESSION**

DREIDIMENSIONALE SCANNERDATENKOMPRIMIERUNG

COMPRESSION DE DONNÉES DE SCANNER TRIDIMENSIONNEL

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**05.09.2012 Bulletin 2012/36**

(73) Proprietor: **Analogic Corporation
Peabody, MA 01960 (US)**

(72) Inventors:
• **DOLAZZA, Enrico
Peabody
MA 01960 (US)**
• **POULO, Louis
Peabody
MA 01960 (US)**
• **ROSHI, Aleksander
Peabody
MA 01960 (US)**

(74) Representative: **Lambsdorff & Lange
Patentanwälte
Partnerschaft mbB
Grillparzerstraße 12A
81675 München (DE)**

(56) References cited:
**WO-A2-2009/073730     US-A1- 2003 076 988
US-A1- 2006 007 766**

• **MANNUDEEP K. KALRA,: "Strategies for CT Radiation Dose Optimization" RADIOLOGY, vol. 230, no. 3, 1 April 2004 (2004-04-01) , pages 619-628, XP002589668 DOI: 10.1148/radiol.2303021726 Retrieved from the Internet: URL:http://radiology.rsna.org/content/230/ 3/619.full.pdf> [retrieved on 2010-06-30]**
• **D. YANG, R. NING, X. ZHANG, R. BETANCOURT, S. LIU: "Image Quality improvement based on Wavelet regularization for Cone beam Breast CT (CBBCT)" MEDICAL IMAGING: IMAGE PROCESSING SPIE, PO BOX 10 BELLINGHAM WA 98227-0010 USA, vol. 7259, no. 725929, 8 February 2009 (2009-02-08), XP040494754**
• **MACQ B ET AL: "Lossless compression for 3D PET" IEEE TRANSACTIONS ON NUCLEAR SCIENCE, IEEE SERVICE CENTER, NEW YORK, NY, US LNKD- DOI:10.1109/23.322948, vol. 41, no. 4, pt.1, 30 October 1993 (1993-10-30), pages 1556-1559, XP002181660 ISSN: 0018-9499**
• **Kenneth Norwich: "Information, Sensation and Perception - Chapter 8: THE ENTROPY OF THE NORMAL DISTRIBUTION", Biopsychology.org, 1 January 2003 (2003-01-01), XP055074306, Retrieved from the Internet: URL:http://www.biopsychology.org/norwich/i sp/isp.htm [retrieved on 2013-08-06]**

## Description

### BACKGROUND

The present application relates to the field of radiography examinations and imaging. It finds particular application with computed tomography (CT) scanners. It also relates to medical, security, and other applications where compressing data would be useful.

CT and other radiography imaging systems are useful to provide information, or images, of interior aspects of an object under examination. Generally, the object is exposed to radiation, and a two-dimensional image and/or a three-dimensional image is formed based upon the radiation absorbed by the interior aspects of the object, or rather an amount of radiation that is able to pass through the object. Typically, highly dense aspects of the object absorb more radiation than less dense aspects, and thus an aspect having a higher density, such as a bone or mass, for example, will be apparent when surrounded by less dense aspects, such as fat tissue or muscle.

A radiation device typically comprises a detector array and a radiation source. In some scanners, such as three-dimensional imaging scanners (e.g., CT scanners), for example, the detector array and radiation source are mounted on opposing sides of a rotating gantry that forms a ring, or donut, around the object under examination. In a conventional CT scanner, the rotating gantry (including the radiation source and/or detector array) is rotated in a circle situated within an x,y plane about an axis extending in the z-dimension (e.g., an "isocenter") during an examination of the object. The object is generally supported by a support article (e.g., a bed, conveyor belt, etc.) that runs substantially parallel to the mechanical center of rotation (e.g., the isocenter). As the rotating gantry is rotated, radiation is substantially continuously emitted from a focal spot of the radiation source.

The detector array is generally comprised of a plurality of pixels, or channels, that detect radiation which impinges the respective pixels or detect energy emitted from the radiation. Typically, the pixels are configured to substantially continuously output an analog signal, and when radiation, or energy, is detected by a pixel, the pixel is configured to emit a pulse in the respective signal indicative of the detected radiation.

The analog signals are typically converted into digital signals (e.g., projection space data that is generally not presented to the human eye) and the digital signals, or the projection space data as it is more commonly referred to, are transmitted in real-time through a data link from the rotating gantry to a component of the radiography system that is substantially stationary, such as an image reconstructor, for example. The required capabilities of the data link generally depend on, among other things, the number of pixels that are emitting signals and/or the rotational speed of the rotating gantry. For example, where the detector array is comprised of approximately twelve thousand pixels, the data link may be required to transmit up to 1.25 Gb/s if sixteen bits are transferred per pixel. Similarly, where the detector array is comprised of approximately two hundred fifty thousand pixels, the data link may be required to transmit up to 20 Gb/s if sixteen bits are transferred per pixel. Data links capable of transferring such large amounts of data per second in real-time are generally costly to manufacture.

There are several reasons why the data is transferred in real-time. For example, examinations of an object by a CT scanner may last for over a minute and may produce large amounts of data (e.g., 1 TB or more of data). It would be difficult and/or costly to produce a high throughput data storage component that has sufficient capacity to store such data and could be practically located on the rotating gantry. Another reason why the data is transferred in real-time is related to the reconstruction process that reconstructs the projection space into image space data that is typically presented to a human. To provide images in a timely manner to an operator (e.g., a doctor, inspector, etc.) during and/or shortly after the examination, some data is generally reconstructed into image space while other projection space data is acquired from detected radiation. Thus, the data is generally transferred while the examination is on-going.

One technique for reducing the amount of data that is required to be transferred through a data-link is data compression. Compression reduces the amount of data transferred through a data link by reducing data redundancy prior to the transfer. In practice it has proven difficult to compress data produced by a CT scanner and other radiography scanners by a significant factor (e.g., by a factor greater than two) because little to none of the data can be lost during compression without diminishing the quality of the images produced (e.g., the loss of data may cause artifacts to appear in the images).

In general, data carrying information (e.g., data comprising information used to reconstruct the images) is highly redundant in both space (e.g., pixel to pixel) and in time (e.g., view to view), and is therefore highly compressible. However, projection space data also comprises uncorrelated, and therefore uncompressible, noise that cannot be separated from the information. Because the noise cannot be separated from the information, the noise makes it difficult to achieve any meaningful, lossless compression (e.g., the data cannot be compressed to a compression ratio of over around 1.5 to two).

US2003/076988 discloses the scale transformation of projection data in computed tomography, so that a signal having standard deviation varying with its mean value is transformed into a signal with a standard deviation not varying with its mean value. US2003/076988 however considers only logarithmic scaling and arctan scaling. Furthermore, scale transformed signals are not subsequently compressed.

Thus, while current techniques and/or systems for transferring data from a rotating gantry to a non-rotating portion of a scanner have proven useful, as CT and other radiography scanners continue to develop and the amount of data produced continues to increase, the cost to transfer the data increases. Therefore, techniques and/or systems that allow large amounts of data to be transferred per unit time (e.g., 1 Gb/s or more) without the loss of data carrying information would be useful.

## SUMMARY

Aspects of the present application address the above matters, and others. According to one aspect, an apparatus is provided. The apparatus comprises a signal pre-processing component configured to prepare an output signal, in projection space and acquired from a CT scan of an object under examination, for compression by remapping the output signal from a first format to a second format, wherein photon noise of the output signal in the second format is substantially constant over a dynamic range. The apparatus also comprises a compression component configured to compress the output signal in the second format.

According to another aspect, a method is provided. The method comprises remapping output signals indicative of a computed tomography (CT) scan of an object under examination from a first format to a second format. The method also comprises compressing the output signals while the output signals are in the second format.

According to another aspect, a method is provided. The method comprises rotating a radiation source from which radiation is emitted with respect to an object under examination and emitting radiation from the radiation source while the radiation source is rotating with respect to the object. The method also comprises detecting emitted radiation that traversed the object and generating an output signal in a first format, the output signal indicative of detected radiation. The method further comprises remapping the output signal from the first format to the second format, wherein the photon noise of the output signal in the second format is substantially constant over a dynamic range. The method also comprises compressing the output signal that is in the second format and uncompressing the compressed output signal.

Those of ordinary skill in the art will appreciate still other aspects of the present application upon reading and understanding the appended description.

## FIGURES

The application is illustrated by way of example and not limitation in the figures of the accompanying drawings, in which like references indicate similar elements and in which:

Fig. 1 is a schematic block diagram illustrating an example scanner.

Fig. 2 illustrates a component block diagram of an example object scanning apparatus.

Fig. 3 illustrates a component block diagram of an example data pre-processing component.

Fig. 4 illustrates a component block diagram of an example decompression component.

Fig. 5 is a flow diagram illustrating an example method of compressing projection space data.

Fig. 6 is an illustration of an example computer-readable medium comprising processor-executable instructions configured to embody one or more of the provisions set forth herein.

## DESCRIPTION

The claimed subject matter is now described with reference to the drawings, wherein like reference numerals are used to refer to like elements throughout. In the following description, for purposes of explanation, numerous specific details are set forth in order to provide a thorough understanding of the claimed subject matter. It may be evident, however, that the claimed subject matter may be practiced without these specific details. In other instances, structures and devices are illustrated in block diagram form in order to facilitate describing the claimed subject matter.

One or more systems and/or techniques for compressing data generated by a CT scanner or other radiography scanner are provided herein. Using such systems and/or techniques, compressed data may be stored on a rotating gantry and/or transferred from the rotating gantry portion of a scanner to a non-rotating portion of the scanner, for example. In this way, storage apparatuses and/or data transfer apparatuses may be produced that are more compact (e.g., so that they can fit within the rotating gantry) and/or cost less to produce, for example, relative to the current storage apparatuses and/or the current data transfer apparatuses on radiography scanners.

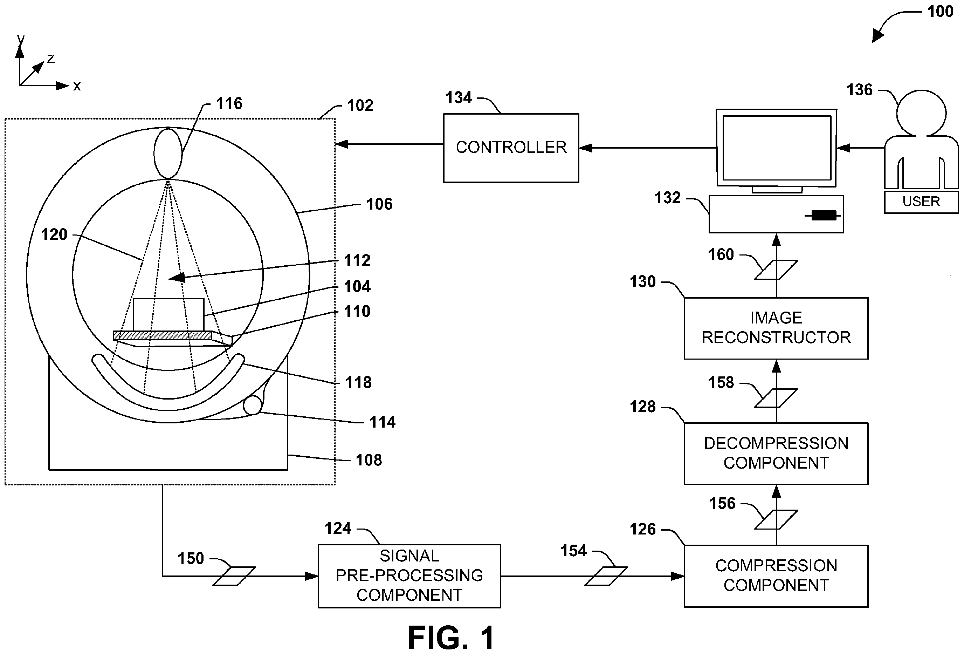

Fig. 1 is an illustration of an example environment 100 in which data that is generated from components comprised within a rotating gantry 106 of a radiography scanner (e.g., a CT scanner) may be compressed and transmitted to components external to the rotating gantry 106 so that one or more images 160 of an object 104 under examination may be produced and viewed by a human user 136, for example. Such a scanner may be used to identify a tumor in a human patient at a medical center and/or to identify potential threats at a security checkpoint, for example.

In the example environment 100, the scanner comprises an object scanning apparatus 102 configured to examine one or more objects 104 (e.g., a series of suitcases at an airport, a human patient, etc.). The object scanning apparatus 102 typically comprises a disk-shaped rotating gantry 106 and a stationary gantry 108. During an examination of the object(s) 104, the object(s) 104 can be placed on a support article 110, such as a bed or conveyor belt, that is selectively positioned in an examination region 112 (e.g., a hollow bore in the rotating gantry portion 106), and the rotating gantry 106 can be rotated about the object(s) 104 by a rotator 114.

The disk-shaped rotating gantry 106 generally surrounds a portion of the examination region 112 and comprises a radiation source 116 (e.g., an ionizing x-ray source) and a detector array 118 that is mounted on a substantially diametrically opposite side of the rotating gantry 106 relative to the radiation source 116.

During an examination of the object(s) 104, the radiation source 116 emits radiation 120 towards the object(s) 104 under examination while the rotating gantry 106 (including the radiation source 116) rotates about the object(s) 104. Generally, in a CT scanner, the radiation 120 is emitted substantially continuously during the examination. However, in some CT scanners and/or in other radiography scanners, the radiation 120 may be emitted intermittently during the rotation.

As the radiation 120 traverses the object(s) 104, the radiation 120 may be attenuated by aspects of the object(s) 104. Because different aspects attenuate different percentages of the radiation 120, an image may be reconstructed based upon the attenuation, or rather the variations in the number of photons that are detected by the detector array 118. For example, more dense aspects of the object(s) 104, such as a bone or metal plate, may attenuate more of the radiation 120 (e.g., causing fewer photons to strike the detector array 118) than less dense aspects, such as skin or clothing.

In some embodiments, while the object(s) 104 is being scanned, or examined, the object(s) 104 may be translated along an axis traveling in the z-dimension (if, as illustrated, the rotating gantry 106 is configured to rotate in an x,y plane). In this way, an object that has a z-dimension greater than the z-dimension of the radiation traversing the object may be scanned more quickly (relative to a step-and-shoot scanning approach). It will be appreciated that if the object(s) 104 is being translated (e.g., in the z direction) during a scan while the rotating gantry 106 is rotating (e.g., in the x,y plane), the scan may be referred to as a helical or spiral scan.

Radiation 120 that impinges the detector array 118 generally creates an electrical charge that may be detected by one or more pixels, or elements, of the detector array 118 that are in close spatial proximity to the location where the radiation impinged. Respective pixels generate an analog signal (in a linear format) indicative of the electrical charge detected. Because the electrical charge detected by the one or more pixels is directly related to the number of photons (e.g., an electrical charge of 1.2 keV may be equivalent to one photon), the output is indicative of the attenuation of the radiation 120 as it traversed the object(s) 104. It will be appreciated that, in one embodiment, when a pixel is not detecting electrical charge, the pixel can emit an analog, baseline signal that indicates that the pixel has detected little to no electrical charge.

It will be understood to those skilled that in some embodiments, an A/D converter (not shown, but generally situated between the object scanning apparatus 102 and the signal pre-processing component 124) may be configured to receive the analog signals and convert the signals into digital signals. The data comprised in the digital signals may be formatted in any one or more of a number of different formats in which the photon noise of the data is dynamic over a dynamic range (e.g., the standard deviation of photon noise varies as a function of the average photon signal). For example, the signals may be converted from an analog, linear format to a digital format, such as 16-bit floating-point format or a quasi-logarithmic format. Data comprised in the digital signals is commonly referred to in the art as projection space data because, like the analog signals, the digital signals are in projection space.

By way of example and not limitation, analog signals that are output by the detector array 118 and/or digitals signals that are yielded from the outputted analog signals may be referred to herein as output signals 150. That is, the terms "output signals" 150 are not intended to be limited to the signals actually output by the detector array, but may also be used to refer to signals that are yielded, derived and/or otherwise calculated or determined from the signals actually output by the detector array.

As an example, a computed tomography (CT) security scanner 100 that includes an x-ray source 116, such as an x-ray tube, can generate x-ray radiation that traverses one or more objects 104, such as a suitcase, traveling from an upstream portion to a downstream portion of an examination region 112 (e.g., moving into or out of the page). In this example, the x-rays that are emitted by the source 116 traverse the examination region 112 that contains the object(s) 104 to be scanned and are detected by an x-ray detector array 118 across from the x-ray source 116. Further a rotator 114, such as a gantry motor drive attached to a rotating gantry portion 106 can be used to rotate the x-ray source 116 and the detector array 118 around the object(s) 104 while the object(s) 104 is translated from the upstream portion of the examination region 112 to the downstream portion, for example. Based upon the amount of energy detected by pixels of the x-ray detector array 118, output signals 150 can be yielded that are indicative of the object(s) 104 under examination.

In the example environment 100, the output signals 150 are transmitted to a signal pre-processing component 124 that is configured to prepare the output signals 150, in projection space, for compression. In one embodiment, configuring the output signals 150 for compression comprises remapping (also referred to herein as converting or encoding) the output signals from a first format to a second format that is more suitable for compression.

The terms "first format" are not intended to be limited to a beginning format (e.g. a format that the output signals 150 are in when immediately generated by pixels and outputted by the detector array 118). Rather, the terms "first format" are used herein in a broad sense to describe any format in which the photon noise is dynamic over a dynamic range (e.g., the standard deviation of the photon noise varies as a function of the average photon signal). Similarly, the terms "second format" are used herein to describe a format in which photon noise is substantially constant over a dynamic range (e.g., the standard deviation of the photon noise does not substantially change as a function of the average photon signal) and not necessarily a second-order format (e.g., a format that the output signals 150 are encode into immediately following the beginning format). A format in which the photon noise is substantially constant over a dynamic range may also be referred to herein as an adaptive format.

It will be understood to those skilled in the art that where the output signals 150 are analog signals, the signal pre-processing component 124 can comprise an A/D converter that converts the output signals 150 (directly) from a (linear) analog signal (e.g., the first format) to a digital signal, wherein the data comprising in the digital signal is in the second format. Such an A/D conversion is unnecessary where the output signals 150 were previously converted into digital signals, so in such an embodiment, the signal pre-processing component 124 can be configured to convert digital output signals 150 from the first format to the second format (e.g., without the use of a second A/D converter). Because the output signals 150 that are received by the signal pre-processing component 124 are digital signals and/or are converted into digital signals by the signal pre-processing component 124, the signals that are output by the signal pre-processing component 124 are digital signals and may be referred to herein as projection space data in the second format 154. That is, the data output by the signal pre-processing component may be referred to herein as either digital signals in the second format 154 or projection space data in the second format 154.

In one embodiment, prior to the remapping, the signal pre-processing component 124 may also be configured to correct the output signals 150 for artifacts introduced into the signals 150 (by the pixels of the detector array 118). For example, the signal pre-processing component 124 can be configured to correct for artifacts by equalizing offset and/or gain variations in the output signals 150 (e.g., similar to adjusting a scale to read "0" before weight is applied).

In the example environment 100, the projection space data in the second format 154 is output from the signal pre-processing component 124 and received by a compression component 126. The compression component 126 is configured to compress the projection space data in the second format 154 into compressed projection space data 156 using compression techniques known to those skilled in the art. In one embodiment, by converting the output signals 150 from a first format to the second format 154 and then compressing the projection space data in the second format 154, the data may be compressed by a factor of 2.5 or more relative to the analog, linear formatted, output signals 150 that were generated by the pixels of the detector array 118. For example, 20 bits in a linear format may be compressed to 5 bits..

The example environment 100 further illustrates a decompression component 128 that is operably coupled to the compression component 126 and is configured to receive the compressed projection space data 156. It will be appreciated that in one embodiment, prior to the decompression component 128 receiving the compressed projection space data 156, the compressed projection space data 156 may pass through a data link (e.g., comprising a transmitter and receiver) (not shown). For example, the data pre-processing component 124 and/or the compression component 126 may be comprised within the rotating gantry 106, and the compressed projection space data 156 may be transferred from the rotating gantry 106 to the decompression component 128 comprised within the stationary gantry 108 through the data link, for example,. Thus, compression prior to transmission can reduce the amount of data required to travel through the data link (e.g., from 16 Gb/s to 4 Gb/s or less).

The decompression component 128 is configured to decompress the compressed projection space data 156 using decompression techniques known to those skilled in the art. The decompression component 128 may also be configured to reformat (e.g., decode and/or encode) the decompressed projection space data 158 into a format suitable for use by an image reconstructor 130, for example, and/or another component of a radiography scanner.

In the example environment 100, decompressed projection space data 158 is transmitted to an image reconstructor 130 configured to receive the decompressed projection space data 158. The image reconstructor 130 is also configured to reconstruct one or more images 160 of the object 104 under examination using analytic, iterative, or other image reconstruction techniques known to those skilled in the art (e.g., 2D filtered back projection). In this way, the data is converted from projection space to image space, a domain that may be more understandable by a user 136 viewing the image(s) 160, for example.

The example environment 100 also includes a terminal 132 (e.g., a computer) configured to receive the image(s) 160, which can be displayed on a monitor of the terminal 132 to a user 136 (e.g., security personnel, medical personnel, etc.). In this way, a user 136 can inspect the image(s) 160 to identify areas of interest within the object(s) 104. The terminal 132 can also be configured to receive user input which can direct the object scanning apparatus 102 how to operate (e.g., a speed to rotate, a speed of a conveyor belt, etc.) and/or can direct the terminal 132 to display an image 160 of the object(s) 104 from a particular angle, for example.

In the example environment 100, a controller 134 is operably coupled to the terminal 132. In one example, the controller 134 is configured to receive user input from the terminal 132 and generate instructions for the object scanning apparatus 102 indicative of operations to be performed. For example, the user 136 may want to rescan the object(s) 104, and the controller 134 may issue an instruction instructing the support article 110 to reverse direction (e.g., bringing the object(s) 104 back into an examination region 112 of the object scanning apparatus 102).

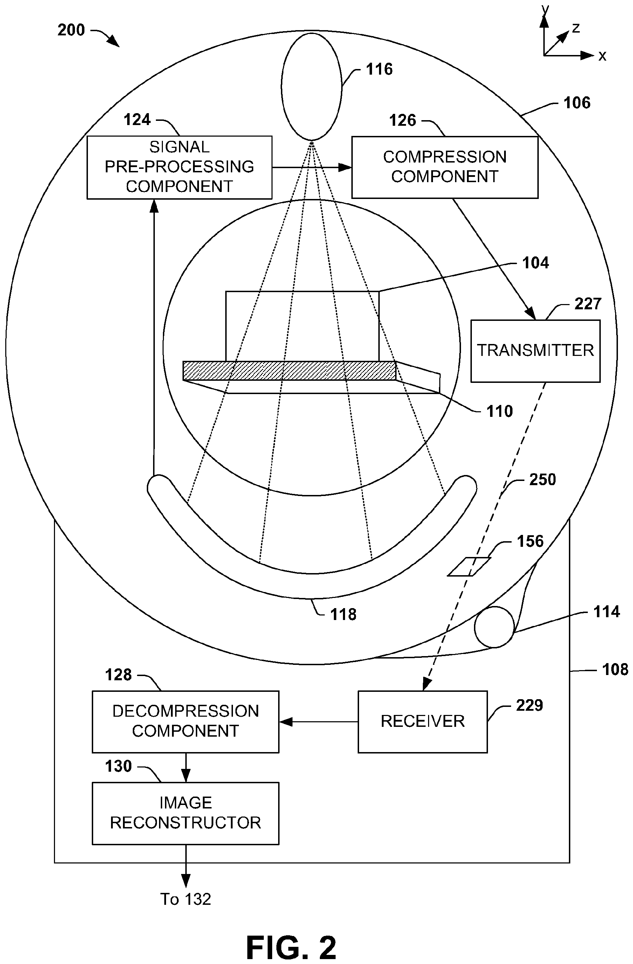

Fig. 2 illustrates an example object scanning apparatus 200 (e.g., 102 in Fig. 1) that may be part of a CT scanner or other radiography scanner, for example. As discussed with respect to Fig. 1, the object scanning apparatus 200 comprises the rotating gantry portion 106 and the stationary gantry portion 108. During an examination of the object(s) 104, situated on the support article 110 in a hollow core of the rotating gantry 106, the rotating gantry 106 is configured to be rotated by a rotating gantry motor 114, for example, about the object(s) 104 with respect to the stationary gantry 108 (which may not rotate). In a one minute examination, for example, the rotating gantry 106 may be configured to complete three or four 360° rotations per second about the object 104. In this way, information may be acquired from a plurality of perspectives (e.g., a side view, a top-down view, etc.), allowing one or more three-dimensional images of an object to be generated, for example.

As illustrated, the rotating gantry 106 can comprise a plurality of components useful for generating an image of interior aspects of the object(s) 104. For example, the radiation source 116 and the detector array 118 are generally comprised within the rotating gantry 106. In the example object scanning apparatus 102, the rotating gantry 106 also comprises the data pre-processing component 124, the compression component 126, and a transmitter 227.

The transmitter 227 may be part of a data link used to transfer the compressed projection space data 156 from the rotating gantry 106 to a non-rotating portion of the scanner (e.g., the stationary gantry 108), for example. In this way, the projection space data may be transmitted in real-time to components of a radiography scanner that are not comprised within the rotating gantry 106, such as the image reconstructor 130, for example. It will be appreciated that because the projection space data is compressed prior to transmission, the amount of data that the data link (e.g., the transmitter 227 and a receiver 229) is required to transfer can be reduced (relative to if the projection space is not compressed) and, thus, may be cheaper to manufacturer. For example, a data link may be required to have the capability of transmitting 4 Gb/s rather than the 16 Gb/s or more that would be required if the projection space data were not compressed.

As illustrated by the dotted line 250, the transmitter 227 is operably coupled to the receiver 229 (e.g., wirelessly or through a slip ring that is conducive to data transfer). The receiver 229 is comprised within a non-rotating part of a radiography scanner. In the illustrated example, the receiver is comprised within the stationary gantry 108.

Once the compressed projection space data 156 is transmitted from the transmitter 227 to the receiver 229 the compressed projection space data 156 may be uncompressed by the decompression component 128 and converted into image space by the image reconstructor 130, for example. In the illustrated example, the decompression component 128 and the image reconstructor 130 are comprised within the stationary gantry 208, but it will be appreciated that in other embodiments, such components may be comprised elsewhere within the radiography scanner, such as in a station nearby the terminal 132, for example.

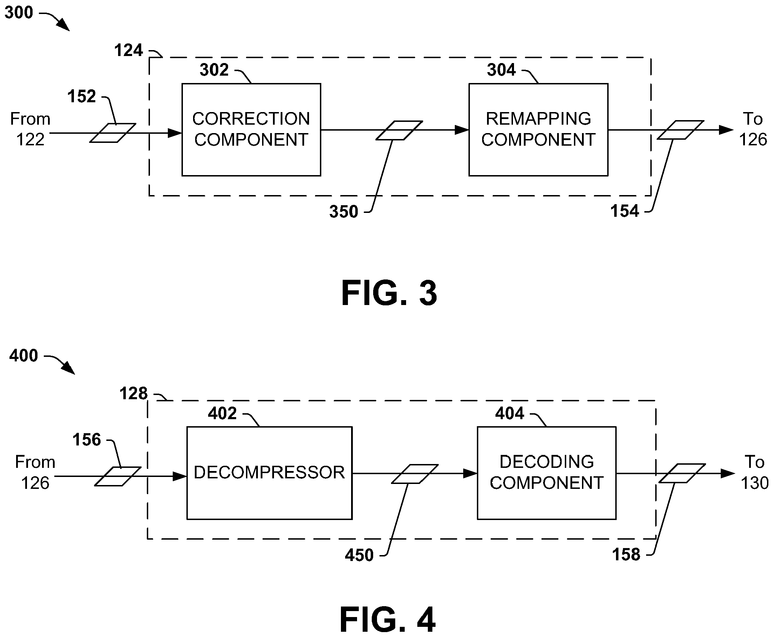

Fig. 3 is a component block diagram of one example embodiment 300 of a signal pre-processing component 124, which can be configured to encode the output signals 150 from a first format to a second format 154. In the second format 154 (e.g., an adaptive format), photon noise is substantially constant over a dynamic range. That is, the standard deviation of the photon noise is substantially constant regardless of the number of photons detected.

As used herein, the first format is defined as a format in which the photon noise of the projection space data varies over a dynamic range. It will be appreciated that the first format is not intended to be limited to the format of the output signals 150 generated by the detector array 118. For example, the output signals 150 may be generated by the detector array 118 in a linear format and converted to a quasi-logarithmic format prior to being received by the signal pre-processing component 124. Thus, as defined above, in this example, the first format could be either the linear format or the quasi-logarithmic format.

In the example embodiment 300, a correction component 302 of the signal pre-processing component 124 corrects for artifacts that may have been introduced into the output signals 150 by the detector array 118, for example. That is, the correction component 302 removes or otherwise alters portions of the signals 150 that are not indicative of radiation, or energy charge, detected by the pixels. For example, the correction component 302 may equalize offset and/or gain variations in the output signals 150 that were introduced by the pixels of the detector array 118. In this way, data that may be perceived by compression algorithms as uncorrelated, but is in fact correlated, may be altered so that the compression algorithms identify the redundancy in the projection space data, for example.

Corrected output signals 350 that are output from the correction component 302 can be transmitted to a remapping component 304 configured to encode the corrected output signals 350, in the first format, into projection space data in the second format 154. Unlike the first format, in the second format the photon noise is substantially constant over the dynamic range. Such a remapping allows the noise entropy of the projection space data to be set at a predetermined level (e.g., a minimum value that preserves information in the projection space data), for example.

Generally, the remapping component comprises memory for storing one or more remapping algorithms and/or a processor for executing the algorithms using the corrected output signals 350. It will be appreciated that in another embodiment, where there is no correction component 302, the algorithms may instead use the uncorrected output signals 150. In one embodiment, the remapping is performed by the remapping component 304 according to the following equations:

$$(1) \qquad \sigma_t = \left(\frac{dt}{ds}\right)\sigma_s$$

where $\sigma t$ is a standard deviation of the signal $t$ in the second format, $\sigma_s$ is the standard deviation of the signal $s$ in the first format presented to the remapping component 304, and the transformation from the signal $s$ to the signal $t$ is constructed so as to result in constant $\sigma_t$ over the dynamic range of the signal $s$.

In an embodiment where $s$ is encoded in a linear format and other noise components are negligible compared to the photon noise (e.g., as is typical in CT), then the signal $s$ and the standard deviation $\sigma_s$ are given by:

$$(2) \qquad s = wn_\gamma\sigma_s$$

$$(3) \qquad \sigma_s = w\sqrt{n_\gamma}$$

where $w$ is a scale factor and $n_\gamma$ is the number of detected photons. The corresponding signal $t$, with constant $\sigma_t$, is given by:

$$(4) \qquad t = 2\sigma_t\sqrt{n_\gamma}.$$

Because the signal output from the detector is generally substantially linear with the number of detected photons $n_\gamma$, equation (4) may be considerate as valid, independent of the format in which the detector output signal 150 is digitized (e.g., FTP, quasi-logarithmic, etc). In other words, the signal $t$ may be independent of the format of the signal presented to the remapping component 304.

Using equation (4) and setting the full scale of $t$ to the value $T$, the full scale number of detected photons to $N_y$, $\eta$ may be defined by :

$$(5) \qquad \sigma_t = \eta\delta_t$$

where $\delta_t$ is the constant quantization interval in the $t$ format.

The resulting number of bits that may be required to adaptively encode the detector output is:

$$(6) \qquad B_t = \log_2(2\eta\sqrt{N_\gamma}) \text{ bits/measurement.}$$

Equation (6) shows that the number of bits that may be required to adaptively encode the output of a CT detector is a function of the number of full scale photons per measurement ($N_y$) and the parameter $\eta$.

In the image domain, a minimum value for $\eta$ in the range of 6-8 is generally required to avoid visible quantization artifacts. However, in projection space, a $\eta$ in the range of 1-2 may be required to avoid visible quantization artifacts in reconstructed images.

As illustrated by the example equations 1-6, the remapping component 304 remaps the corrected projection space data 350 (or uncorrected projection space data if the correction component 302 does not exists) into a format in which the photon noise of the projection space is substantially constant over a dynamic range.

The remapping component 304 is configured to output the projection space data in the second format 154. A compression component (e.g., 126 in Fig. 1) can be configured to receive the projection space data in the second format 154 and to compress the data using lossless data compression techniques or other compression techniques known to those skilled in the art.

The function of lossless compression is reducing or minimizing the number of bits necessary to encode the signal information. The lower bound on this number is generally the entropy of the data set to be encoded, for example, which includes the entropy of the underlying information content of the signal and the entropy of the noise on the signal. Efficient data compression algorithms can achieve results which are typically only slightly higher (e.g., within a few percent) of this theoretical lower bound. By way of example and not limitation, an average encoded and compressed word length can be calculated or derived as follows.

If the noise and signal components are independent of each other, then the entropy $H$ of the combined signal and noise in the measurements may be given by:

$$(7) \qquad H(total) = H(signal\ information) + H(noise)$$

It will be appreciated that it is known to those skilled in the art that the entropy of the signal information is low (e.g., on the order of one bit/measurement) in CT. Assuming the photon noise has a Gaussian distribution, in adaptive domain (e.g., the second format), the noise entropy $H(n)$ is given by:

$$(8) \qquad H(noise) = \log_2(n\sqrt{2\pi e}) \approx \log_2 \eta + 2.$$

Thus, an effective compression scheme can realize an average encoded word length that is substantially the theoretical minimum. Assuming a 10% compression "inefficiency," readily achieved in practice, adaptively encoded (e.g., projection space data in the second format) can achieve an average word length *Navg*, calculated as follows:

$$(9) \qquad \log_2 \eta + 2 = 2\ for\ \eta = 1,$$

$$Navg = 1.1\big(H(signal\ information) + H(noise)\big)$$

$$Navg = 1.1(1 + 2) = 3.3\ bits/measurement.$$

The result is that the average encoded and compressed word length may be on the order of 3.3 bits per measurement. In other examples, the average encoded and compressed word length may be greater or less than 3.3 bits per measurement depending upon the efficiency of the compression technique used by the compression component, for example.

Fig. 4 is a component block diagram of one example 400 of a decompression component 128, which can be configured to decompress compressed projection space data 156 transmitted to it (through a data link) from the compression component 126.

In the example 400, the decompression component 128 comprises a decompressor 402 configured to decompress the compressed projection space data 156 using decompression techniques known to those skilled in the art. In this way, the projection space data may be decompressed such that the projection space data in the second format 154 appears as it would have appeared prior to compression.

The example 400 also comprises a decoding component 404 configured to decode the decompressed projection space data 450 into a format similar to the first format and/or encode the projection space data into yet another format that is more suitable to be received by other components of the radiography scanner, such as the image reconstructor 130, for example, using suitable analytic, iterative, or other decoding/encoding techniques known to those skilled in the art.

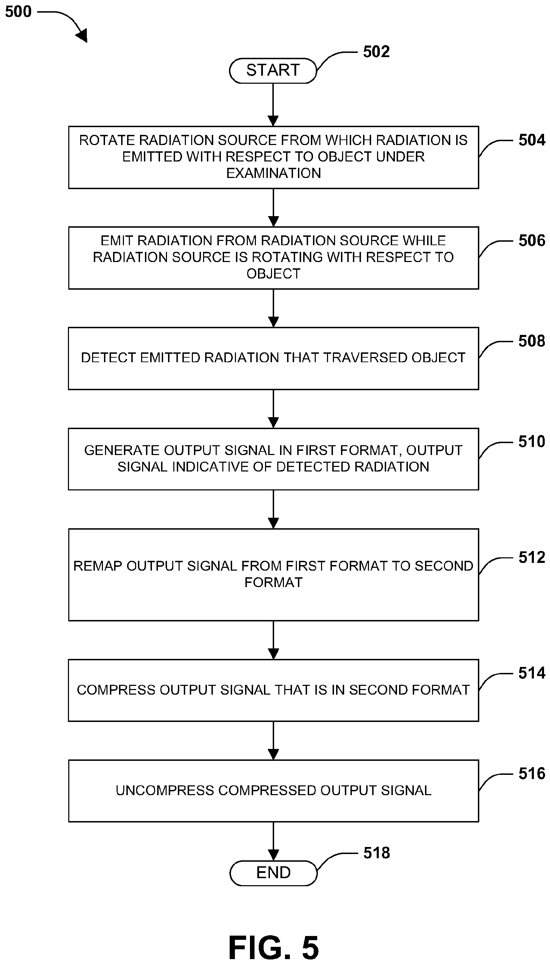

Fig. 5 illustrates an example method 500 for compressing output signals (e.g., 150 in Fig. 1). Such signals may be generated by a CT scanner and/or other radiography scanners, for example. In this way, signals, or rather data that comprises the signals, may be transferred from a rotating gantry portion of the scanner to a non-rotating portion of a scanner in a compressed manner, reducing the amount of data that is transferred in real-time, for example.

The example method 500 begins at 502, and a radiation source from which radiation is emitted is rotated with respect to the object under examination at 504. For example, the radiation source may follow a semicircular or circular path in an x,y plane. It will be appreciated that, generally, the radiation source is comprised within a rotating gantry (e.g. a donut-like structure), and thus the radiation source is rotated when a rotating gantry is rotated (by a rotating gantry motor).

At 506, radiation is emitted from the radiation source while the radiation source is rotating with respect to the object. That is, the object is positioned at about an axis of rotation (e.g., an isocenter), and the radiation source rotates about the object and emits radiation. It will be appreciated that the radiation source may emit radiation substantially continuously and/or may emit radiation intermittently during the rotation. In a typical CT scanner, the radiation is emitted substantially continuously as the radiation source is rotated. By emitting radiation while the rotating gantry is rotating, radiation can be emitted from a plurality of angles with respect to the object (e.g., so that one or more three-dimensional images of an object can be generated from detected radiation).

At 508, emitted radiation that traversed the object is detected. Generally, the radiation is detected by a detector array also comprised within the rotating gantry. Thus, both the detector array and the radiation source are rotating (in unison) about the object under examination.

The detector array is generally comprised of a plurality of pixels, or elements, that are configured to detect electric charge that is emitted when the radiation impinges the detector array. At 510, output signals in a first format are generated based upon the detected electric charge. As used herein, the output signals may be analog signals that are produced by the pixels when electric charge is detected and/or digital signals that are produced from the analog signals (e.g., by an A/D converter). As used herein, the first format may be any one or more of the numerous formats that are known to those skilled in the art in which photon noise of the output signals varies over a dynamic range (e.g., the noise has a standard deviation that fluctuates based upon the number of photons detected). Thus, the standard deviation of noise may be greater when the radiation is traveling through a thin portion of the object (e.g., traveling from the frontside to the back-side of a human) than when it is traveling through a thicker portion of the object (e.g., traveling from shoulder to shoulder on a human).

It will be appreciated that the terms "first format" are not intended to necessarily describe the beginning format of the output signals. For example, the output signals may begin in a linear format and then the output signals may be converted into a digital signal and encoded in a quasi-logarithmic format or a 16-bit floating format, for example. Either of these formats (e.g., the beginning format or the "second-ordered" format) may be referred to as the first format because in either format, the photon noise varies over a dynamic range.

At 512, the output signals are remapped from the first format to a second format. While encoded in the second format, the photon noise of the output signals is substantially constant over a dynamic range. That is, the signals are encoded into a format wherein the standard deviation of the noise does not change (regardless of the number of photons detected). It will be appreciated that such a format may be referred to as an adaptive format.

It will be understood to those skilled in the art that were the output signals in the first format are analog signals, the output signals may be converted to digital signals while the output signals are being encoded into the second format. That is, the output signals may be converted by an A/D converter from analog signals to digital signals substantially at the same time as the format of the signals are changing from the first format to the second format (e.g., an A/D converter may be configured to convert the analog signals into digital signals having an adaptive format). Because the output signals in the second format are digital signals, the output signals can also be referred to as projection space data in the second format.

Once the projection space data is encoded in the second format, characteristics of the projection space data may be adjusted based upon predetermined criteria. For example, a noise entropy of the projection space data may be adjusted to a predetermined level. In one example, the noise entropy is adjusted to a minimum value that still preserves the information comprised within the projection space data in the second format. Those of ordinary skill in the art will appreciate that the minimum value of the noise entropy that preserves information is calculable and may be a function of the current supplied to the radiation source and/or the types of objects that are generally examined, for example. By reducing the noise entropy, the uncertainty within the projection space data can be reduced (e.g., creating more redundancy in the data and thus allowing for more data compression).

At 514, the output signals in the second format are compressed using analytical, iterative, or other compression techniques known to those skilled in the art. In this way, redundancy in the data is combined (e.g., reducing the total size of the projection space data). In one embodiment, where the first format is a linear format, the projection space data may be compressed to a factor of 2.5 or more relative to the amount of the projection space data in the first format. For example, 16 Gb of projection space data may be reduced to 6.4 Gb of projection space data or less.

It will be appreciated that by compressing the data, the amount of data that is transferred (e.g., per second in real-time via a data link) from one portion of a radiography scanner to another portion of the radiography scanner may be reduced (relative to the amount transferred if there is no compression of the projection space data). For example, where the radiography scanner comprises a rotating gantry, such as a rotating gantry on a CT scanner, for example, the compressed data may be transmitted from the rotating gantry portion of the scanner and received on a non-rotating portion of the scanner. In this way, the data may be reconstructed into image space and/or displayed on a monitor for human observation, for example.

It will be appreciated that while such transfers of data may occur without compression, compression reduces the amount of data that is transferred. However, by reducing the amount of data that is transferred, the data link can be obtained at a lower cost and/or the data can be stored more compactly (in the rotating gantry and/or in a storage component on a non-rotating portion of the scanner) than it could be in a non-compressed form, for example.

At 516, the compressed output signals are uncompressed using techniques known to those skilled in the art. It will be appreciated that the projection space data in the second format may also be encoded into another format (e.g., a format more suitable for image reconstruction) and/or decoded back into the first format. In this way, the data may be encoded/decoded into a format that may more useable (than the second format) by components of the radiography scanner

The method 500 ends at 518.

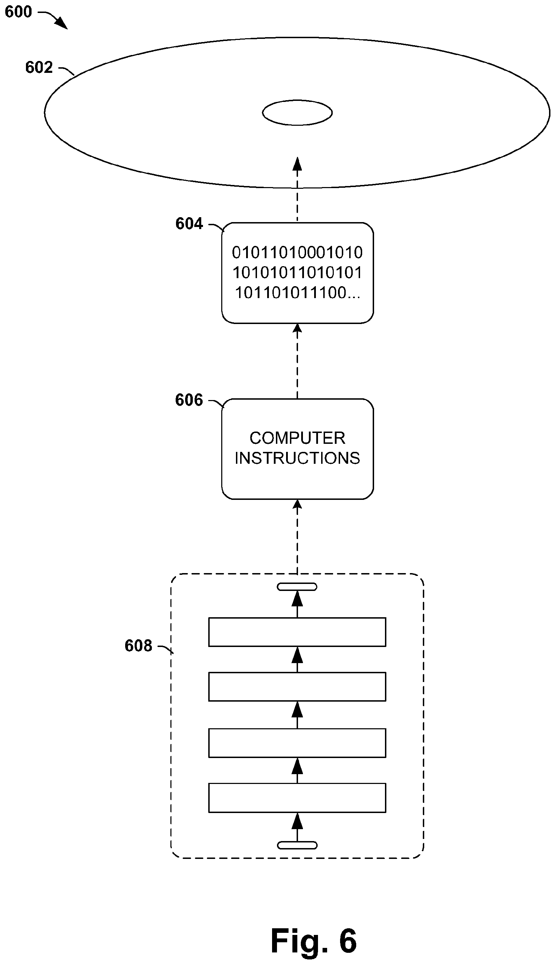

Still another embodiment involves a computer-readable medium comprising processor-executable instructions configured to implement one or more of the techniques presented herein. An example computer-readable medium that may be devised in these ways is illustrated in Fig. 6, wherein the implementation 600 comprises a computer-readable medium 602 (e.g., a CD-R, DVD-R, or a platter of a hard disk drive), on which is encoded computer-readable data 604. This computer-readable data 604 in turn comprises a set of computer instructions 606 configured to operate according to one or more of the principles set forth herein. In one such embodiment 600, the processor-executable instructions 606 may be configured to perform a method 608, such as the example method 500 of Fig.5, for example. In another example the processor-executable instructions 606 may be configured to implement a system, such as at least some of the exemplary scanner 100 of Fig. 1, for example. Many such computer-readable media may be devised by those of ordinary skill in the art that are configured to operate in accordance with one or more of the techniques presented herein.

It will be appreciated that there are numerous benefits to the systems and/or techniques described herein. For example, the compression can be lossless so techniques and/or systems can be used in applications where it is important that the projection space data is preserved (e.g., CT applications). Additionally, the high compression ratio (relative to other lossless compression techniques used on projection space data) reduces the required capability of the data-link (e.g., wireless transmitter/receiver, slip-ring, etc.) and/or the required capabilities of a data storage component (if present in a scanner), for example. Additionally, the compression may be implemented in hardware or software based upon the application and/or the desires of a user, for example.

Moreover, the words "example" and/or "exemplary" are used herein to mean serving as an example, instance, or illustration. Any aspect, design, etc. described herein as "example" and/or "exemplary" is not necessarily to be construed as advantageous over other aspects, designs, etc. Rather, use of these terms is intended to present concepts in a concrete fashion. As used in this application, the term "or" is intended to mean an inclusive "or" rather than an exclusive "or". That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. In addition, the articles "a" and "an" as used in this application and the appended claims may generally be construed to mean "one or more" unless specified otherwise or clear from context to be directed to a singular form.

The invention is defined in the appended claims. Also, although the disclosure has been shown and described with respect to one or more implementations, equivalent alterations and modifications will occur to others skilled in the art based upon a reading and understanding of this specification and the annexed drawings. The disclosure includes all such modifications and alterations and is limited only by the scope of the following claims. In particular regard to the various functions performed by the above described components (e.g., elements, resources, etc.), the terms used to describe such components are intended to correspond, unless otherwise indicated, to any component which performs the specified function of the described component (*e.g.*, that is functionally equivalent), even though not structurally equivalent to the disclosed structure which performs the function in the herein illustrated example implementations of the disclosure. In addition, while a particular feature of the disclosure may have been disclosed with respect to only one of several implementations, such feature may be combined with one or more other features of the other implementations as may be desired and advantageous for any given or particular application. Furthermore, to the extent that the terms "includes", "having", "has", "with", or variants thereof are used in either the detailed description or the claims, such terms are intended to be inclusive in a manner similar to the term "comprising."

## Claims

1. An apparatus (100), comprising:

a signal pre-processing component (124) configured to prepare an output signal, in projection space and acquired from a CT scan of an object (104) under examination, for compression by remapping the output signal from a first format to a second format,
wherein the first format is a linear format,
wherein the output signal s in the first format is given by $s = wn_\gamma \sigma_s$, wherein $w$ is a scale factor, $n_\gamma$ is the number

of detected photons acquired from the CT scan, wherein the detected photons are emitted by a radiation source and traversed the object (104), and $\sigma_s$ is the standard deviation of the signal s, wherein the standard deviation $\sigma_s$ is given by $\sigma_s = w\sqrt{n_\gamma}$,

wherein the output signal t in the second format is given by $t = 2\sigma_t\sqrt{n_\gamma}$, wherein $\sigma_t$ is the standard deviation of the signal t,

wherein $\sigma_s$ is constant,

wherein the remapping of the output signal from the first format to the second format is performed according to

$$\sigma_t = \left(\frac{dt}{ds}\right)\sigma_s,$$

wherein photon noise of the output signal in the second format is constant over a dynamic range, wherein the standard deviation of the photon noise does not change as a function of the average photon signal; and a compression component (126) configured to compress the output signal in the second format.

2. The apparatus of claim 1, wherein the signal pre-processing component (124) corrects the output signal in the first format by equalizing at least one of an offset and a gain variation in the output signal.

3. A method (500) comprising:

remapping (512) output signals indicative of a computed tomography (CT) scan of an object (104) under examination from a first format to a second format,
wherein the first format is a linear format,
wherein the output signal s in the first format is given by $s = wn_\gamma\sigma_s$, wherein $w$ is a scale factor, $n_\gamma$ is the number of detected photons acquired from the CT scan, wherein the detected photons are emitted by a radiation source and traversed the object (104), and $\sigma_s$ is the standard deviation of the signal s, wherein the standard deviation $\sigma_s$ is given by $\sigma_s = w\sqrt{n_\gamma}$,

wherein the output signal t in the second format is given by $t = 2\sigma_t\sqrt{n_\gamma}$, wherein $\sigma_t$ is the standard deviation of the signal t,
wherein $\sigma_t$ is constant,
wherein the remapping of the output signal from the first format to the second format is performed according to

$$\sigma_t = \left(\frac{dt}{ds}\right)\sigma_s;$$

wherein photon noise of the output signal in the second format is constant over a dynamic range, wherein the standard deviation of the photon noise does not change as a function of the average photon signal; and compressing (514) the output signals while the output signals are in the second format.

4. The method of claim 3, wherein the noise entropy is adjusted to a minimum value that preserves information in the output signals in the second format.

5. A computer readable medium comprising instructions that when executed via a processing unit perform a method, the method (500) comprising:

rotating (504) a radiation source from which radiation is emitted with respect to an object (104) under examination;
emitting (506) radiation from the radiation source while the radiation source is rotating with respect to the object;
detecting (508) emitted radiation that traversed the object (104);
generating (510) an output signal in a first format, the output signal indicative of the detected radiation;
remapping (512) the output signal from the first format to a second format,
wherein the first format is a linear format,
wherein the output signal s in the first format is given by $s = wn_\gamma\sigma$, wherein $w$ is a scale factor, $n_\gamma$ is the number of detected photons acquired from the CT scan, wherein the detected photons are emitted by the radiation source and traversed the object (104), and $\sigma_s$ is the standard deviation of the signal s,
wherein the standard deviation $\sigma_s$ is given by $\sigma_s = w\sqrt{n_\gamma}$,

wherein the output signal t in the second format is given by $t = 2\sigma_t\sqrt{n_\gamma}$, wherein $\sigma_t$ is the standard deviation of the signal t,

wherein $\sigma_t$ is constant,

wherein the remapping of the output signal from the first format to the second format is performed according to

$$\sigma_t = \left(\frac{dt}{ds}\right)\sigma_s,$$

wherein photon noise of the output signal in the second format is constant over a dynamic range, wherein the standard deviation of the photon noise does not change as a function of the average photon signal;

compressing (514) the output signal that is in the second format; and

uncompressing (516) the compressed output signal.

6. The computer readable medium of claim 5, the method comprising, before compressing, adjusting a noise entropy of the output signal in the second format based upon predetermined criteria.

7. The computer readable medium of claim 5, the method comprising, before uncompressing:
transmitting the compressed output signal from a rotating gantry portion of a computed tomography (CT) scanner to a non-rotating portion of the CT scanner.

**Patentansprüche**

1. Vorrichtung (100), umfassend:

eine Signal-Vorverarbeitungskomponente (124), dazu ausgelegt, ein Ausgabesignal vorzubereiten, im Projektionsraum und von einem CT-Scan eines zu untersuchenden Objekts (104) erhalten, für eine Komprimierung durch ein Remapping des Ausgabesignals von einem ersten Format in ein zweites Format,
wobei das erste Format ein lineares Format ist,
wobei das Ausgabesignal s in dem ersten Format durch $s = wn_\gamma\sigma_s$ gegeben ist, wobei $w$ ein Skalierungsfaktor ist, $n_\gamma$ ist die Anzahl detektierter Photonen, erhalten durch den CT-Scan, wobei die detektierten Photonen durch eine Strahlungsquelle emittiert werden und das Objekt (104) durchquert haben, und $\sigma_s$ ist die Standardabweichung des Signals s,

wobei die Standardabweichung $\sigma_s$ durch $\sigma_s = w\sqrt{n_\gamma}$ gegeben ist,

wobei das Ausgabesignal t in dem zweiten Format durch $t = 2\sigma_t\sqrt{n_\gamma}$ gegeben ist, wobei $\sigma_t$ die Standardabweichung des Signals t ist,
wobei $\sigma_t$ konstant ist,

wobei das Remapping des Ausgabesignals von dem ersten Format in das zweite Format gemäß $\sigma_t = \left(\frac{dt}{ds}\right)\sigma_s$

durchgeführt wird,
wobei ein Photonen-Rauschen des Ausgabesignals in dem zweiten Format konstant ist über einen dynamischen Bereich, wobei sich die Standardabweichung des Photonen-Rauschens als eine Funktion des Durchschnitts-Photonen-Signals nicht ändert; und
eine Komprimierungskomponente (126), dazu ausgelegt, das Ausgabesignal in das zweite Format zu komprimieren.

2. Vorrichtung nach Anspruch 1, wobei die Signal-Vorverarbeitungskomponente (124) das Ausgabesignal in dem ersten Format korrigiert durch Entzerren von mindestens einem von einem Offset und einer Verstärkungsvariation in dem Ausgabesignal.

3. Verfahren (500), umfassend:

Remapping (512) von Ausgabesignalen, bezeichnend für einen Computertomographie (CT)-Scan eines zu untersuchenden Objekts (104), von einem ersten Format in ein zweites Format,
wobei das erste Format ein lineares Format ist,

wobei das Ausgabesignal s in dem ersten Format durch $s = wn_\gamma\sigma_s$ gegeben ist, wobei $w$ ein Skalierungsfaktor ist, $n_\gamma$ ist die Anzahl detektierter Photonen, erhalten durch den CT-Scan, wobei die detektierten Photonen von einer Strahlungsquelle emittiert werden und das Objekt (104) durchquert haben, und $\sigma_s$ ist die Standardabweichung des Signals s,

wobei die Standardabweichung $\sigma_s$ durch $\sigma_s = w\sqrt{n_\gamma}$ gegeben ist,

wobei das Ausgabesignal t in dem zweiten Format durch $t = 2\sigma_t\sqrt{n_\gamma}$ gegeben ist, wobei $\sigma_t$ die Standardabweichung des Signals t ist,

wobei $\sigma_t$ konstant ist,

wobei das Remapping des Ausgabesignals von dem ersten Format in das zweite Format gemäß

$$\sigma_t = \left(\frac{dt}{ds}\right)\sigma_s$$ durchgeführt wird;

wobei ein Photonen-Rauschen des Ausgabesignals in dem zweiten Format konstant ist über einen dynamischen Bereich, wobei sich die Standardabweichung des Photonen-Rauschens als eine Funktion des Durchschnitts-Photonen-Signals nicht ändert; und

Komprimieren (514) des Ausgabesignals, während das Ausgabesignal in dem zweiten Format ist.

4. Verfahren nach Anspruch 3, wobei die Rausch-Entropie auf einen Minimalwert eingestellt wird, welcher Information in dem Ausgabesignal in dem zweiten Format erhält.

5. Computerlesbares Medium, umfassend Befehle, welche, wenn über eine Verarbeitungseinheit ausgeführt, ein Verfahren durchführen, das Verfahren (500) umfassend:

Rotieren (504) einer Strahlungsquelle, von welcher Strahlung emittiert wird in Bezug auf ein zu untersuchendes Objekt (104);

Emittieren (506) von Strahlung von der Strahlungsquelle, während die Strahlungsquelle rotiert in Bezug auf das Objekt;

Detektieren (508) emittierter Strahlung, welche das Objekt (104) durchquert hat;

Erzeugen (510) eines Ausgabesignals in einem ersten Format, wobei das Ausgabesignal bezeichnend für die detektierte Strahlung ist;

Remapping (512) des Ausgabesignals von dem ersten Format in ein zweites Format,

wobei das erste Format ein lineares Format ist,

wobei das Ausgabesignal s in dem ersten Format durch $s = wn_\gamma\sigma_s$ gegeben wird, wobei $w$ ein Skalierungsfaktor ist, $n_\gamma$ ist die Anzahl detektierter Photonen, erhalten durch den CT-Scan, wobei die detektierten Photonen von der Strahlungsquelle emittiert werden und das Objekt (104) durchquert haben, und $\sigma_s$ ist die Standardabweichung des Signals s,

wobei die Standardabweichung $\sigma_s$ durch $\sigma_s = w\sqrt{n_\gamma}$ gegeben ist,

wobei das Ausgabesignal t in dem zweiten Format durch $t = 2\sigma_t\sqrt{n_\gamma}$ gegeben ist, wobei $\sigma_t$ die Standardabweichung des Signals t ist,

wobei $\sigma_t$ konstant ist,

wobei das Remapping des Ausgabesignals von dem ersten Format in das zweite Format gemäß $\sigma_t = \left(\frac{dt}{ds}\right)\sigma_s$

durchgeführt wird,

wobei ein Photonen-Rauschen des Ausgabesignals in dem zweiten Format über einen dynamischen Bereich konstant ist, wobei sich die Standardabweichung des Photonen-Rauschens als eine Funktion des Durchschnitts-Photonen-Signals nicht ändert;

Komprimieren (514) des Ausgabesignals, welches in dem zweiten Format ist; und

Dekomprimieren (516) des komprimierten Ausgabesignals.

6. Computerlesbares Medium nach Anspruch 5, das Verfahren umfassend, vor dem Komprimieren, Einstellen einer Rausch-Entropie des Ausgabesignals in dem zweiten Format basierend auf vorbestimmten Kriterien.

7. Computerlesbares Medium nach Anspruch 5, das Verfahren umfassend, vor dem Dekomprimieren:
Übertragen des komprimierten Ausgabesignals von einem rotierenden Gantry-Teil eines Computertomographie (CT)-Scanners zu einem nichtrotierenden Teil des CT-Scanners.

**Revendications**

1.  Appareil (100) comprenant :

    un élément de prétraitement de signal (124) configuré pour préparer un signal de sortie, dans un espace de projection et acquis à partir d'une tomodensitométrie d'un objet (104) en train d'être examiné, pour la compression par recartographie du signal de sortie d'un premier format à un deuxième format,
    dans lequel le premier format est un format linéaire,
    dans lequel le signal de sortie s dans le premier format est donné par $s = wn_y\sigma_s$, dans lequel $w$ est un facteur d'échelle, $n_y$ est le nombre de photons détectés acquis d'après la tomodensitométrie, dans lequel les photons détectés sont émis par une source de rayonnement et ont traversé l'objet (104), et $\sigma_s$ est l'écart type du signal $s$,

    dans lequel l'écart type $\sigma_s$ est donné par $\sigma_s = w\sqrt{n_y}$, dans lequel le signal de sortie t au deuxième format est donné par $t = 2\sigma_t\sqrt{n_y}$, dans lequel $\sigma_t$ est l'écart type du signal t,

    dans lequel $\sigma_t$ est constant,
    dans lequel la recartographie du signal de sortie du premier format au deuxième format est réalisée selon

    $$\sigma_t = \left(\frac{dt}{ds}\right)\sigma_s,$$

    dans lequel du bruit photonique du signal de sortie au deuxième format est constant sur une plage dynamique, dans lequel l'écart type du bruit photonique ne change pas en tant qu'une fonction du signal à photons en moyenne ; et
    un élément de compression (126) configuré pour compresser le signal de sortie au deuxième format.

2.  Appareil selon la revendication 1, dans lequel l'élément de prétraitement de signal (124) corrige le signal de sortie au premier format en égalisant au moins l'un/une parmi un décalage et une variation de gain dans le signal de sortie.

3.  Procédé (500) comprenant :

    la recartographie (512) de signaux de sortie indicatifs d'une tomodensitométrie (CT) d'un objet (104) en train d'être examiné d'un premier format à un deuxième format,
    dans lequel le premier format est un format linéaire,
    dans lequel le signal de sortie s dans le premier format est donné par $s = wn_y\sigma_s$, dans lequel $w$ est un facteur d'échelle, $n_y$ est le nombre de photons détectés acquis d'après la tomodensitométrie, dans lequel les photons détectés sont émis par une source de rayonnement et ont traversé l'objet (104), et $\sigma_s$ est l'écart type du signal $s$,

    dans lequel l'écart type $\sigma_s$ est donné par $\sigma_s = w\sqrt{n_y}$, dans lequel le signal de sortie t au deuxième format est donné par $t = 2\sigma_t\sqrt{n_y}$, dans lequel $\sigma_t$ est l'écart type du signal t,

    dans lequel $\sigma_t$ est constant,
    dans lequel la recartographie du signal de sortie du premier format au deuxième format est réalisée selon

    $$\sigma_t = \left(\frac{dt}{ds}\right)\sigma_s,$$

    dans lequel du bruit photonique du signal de sortie au deuxième format est constant sur une plage dynamique, dans lequel l'écart type du bruit photonique ne change pas en tant qu'une fonction du signal à photons en moyenne ; et
    la compression (514) des signaux de sortie pendant que les signaux de sortie sont au deuxième format.

4.  Procédé selon la revendication 3, dans lequel l'entropie du bruit est ajustée à une valeur minimale qui préserve l'information dans les signaux de sortie au deuxième format.

**5.** Support lisible par informatique comprenant des instructions qui, lorsque exécutées via une unité de traitement, mettent en oeuvre un procédé, le procédé (500) comprenant :

la mise en rotation (504) d'une source de rayonnement à partir de laquelle un rayonnement est émis par rapport à un objet (104) en train d'être examiné ;

l'émission (506) d'un rayonnement à partir de la source de rayonnement pendant que l'on fait tourner la source de rayonnement par rapport à l'objet ;

la détection (508) du rayonnement émis qui a traversé l'objet (104) ;

la génération (510) d'un signal de sortie dans un premier format, le signal de sortie étant indicatif du rayonnement détecté ;

la recartographie (512) du signal de sortie du premier format à un deuxième format,

dans lequel le premier format est un format linéaire,

dans lequel le signal de sortie s dans le premier format est donné par $s = wn_y\sigma_s$, dans lequel $w$ est un facteur d'échelle, $n_y$ est le nombre de photons détectés acquis d'après la tomodensitométrie, dans lequel les photons détectés sont émis par la source de rayonnement et ont traversé l'objet (104), et $\sigma_s$ est l'écart type du signal $s$,

dans lequel l'écart type $\sigma_s$ est donné par $\sigma_s = w\sqrt{n_\gamma}$, dans lequel le signal de sortie t au deuxième format est donné par $t = 2\sigma_t\sqrt{n_\gamma}$, dans lequel $\sigma_t$ est l'écart type du signal t,

dans lequel $\sigma_t$ est constant,

dans lequel la recartographie du signal de sortie du premier format au deuxième format est réalisée selon

$$\sigma_t = \left(\frac{dt}{ds}\right)\sigma_s,$$

dans lequel du bruit photonique du signal de sortie au deuxième format est constant sur une plage dynamique, dans lequel l'écart type du bruit photonique ne change pas en tant qu'une fonction du signal à photons en moyenne ;

la compression (514) du signal de sortie qui est au deuxième format ; et

la décompression (516) du signal de sortie compressé.

**6.** Support lisible par informatique selon la revendication 5, le procédé comprenant, avant la compression, l'ajustement d'une entropie de bruit du signal de sortie au deuxième format sur la base de critères prédéterminés.

**7.** Support lisible par informatique selon la revendication 5, le procédé comprenant, avant la décompression :
la transmission du signal de sortie compressé d'une partie de portique rotative d'un scanner de tomodensitométrie (CT) à une partie non rotative du scanner de tomodensitométrie.

**FIG. 1**

EP 2 493 382 B1

**FIG. 2**

**FIG. 3**

**FIG. 4**

500 —

502 —

( START )

ROTATE RADIATION SOURCE FROM WHICH RADIATION IS EMITTED WITH RESPECT TO OBJECT UNDER EXAMINATION — 504

EMIT RADIATION FROM RADIATION SOURCE WHILE RADIATION SOURCE IS ROTATING WITH RESPECT TO OBJECT — 506

DETECT EMITTED RADIATION THAT TRAVERSED OBJECT — 508

GENERATE OUTPUT SIGNAL IN FIRST FORMAT, OUTPUT SIGNAL INDICATIVE OF DETECTED RADIATION — 510

REMAP OUTPUT SIGNAL FROM FIRST FORMAT TO SECOND FORMAT — 512

COMPRESS OUTPUT SIGNAL THAT IS IN SECOND FORMAT — 514

UNCOMPRESS COMPRESSED OUTPUT SIGNAL — 516

( END ) — 518

FIG. 5

600

602

604

01011010001010
10101011010101
101101011100...

606

COMPUTER
INSTRUCTIONS

608

# Fig. 6

**REFERENCES CITED IN THE DESCRIPTION**

**Patent documents cited in the description**

- US 2003076988 A